# FASCICULE DE BREVET EUROPEEN

(11) **EP 0 461 096 B1**
(45) Date de publication et mention de la délivrance du brevet: **05.02.1997**
(21) Numéro de dépôt: 91870086.5
(22) Date de dépôt: 31.05.1991
(51) Int. Cl.: C07D 207/444, C07D 207/448

(54) **Procédé de fabrication de N-phénylmaléimide**
Verfahren zur Herstellung von N-Phenylmaleinimid
Process for the production of N-phenylmaleimide

(30) Priorité: 05.06.1990 GB 9012453
(43) Date de publication de la demande: 11.12.1991
(73) Titulaire: U C B, S.A., B-1050 Bruxelles (BE)
(72) Inventeur: Van Gysel, August, B-1710 Dilbeek (BE); Vanden Eynde, Ivan, B-2850 Keerbergen (BE); Vanovervelt, Jean-Claude, B-7502 Warchin (BE)
(74) Mandataire: Dusseldorp, Raymond

(56) Documents cités:
- EP-A- 0 129 125
- EP-A- 0 165 574
- EP-A- 0 213 933
- EP-A- 0 334 497
- EP-A- 2 649 743
- GB-A- 2 043 054
- PATENT ABSTRACTS OF JAPAN, vol. 9, no. 260 (C-309)[1983], 17 octobre 1983; & JP-A-60 112 758 (TORAY K.K.) 19-06-1985
- PATENT ABSTRACTS OF JAPAN, vol. 9, no. 260 (C-309)[1983], 17 octobre 1983; & JP-A-60 112 759 (TORAY K.K.) 19-06-1985
- PATENT ABSTRACTS OF JAPAN, vol. 11, no. 262 (C-442)[2709], 25 août 1987; & JP-A-62 63 561 (NIPPON SHOKUBAI KAGAKU KOGYO CO., LTD) 20-03-1987

## Description

La présente invention se rapporte à un nouveau procédé de fabrication de N-phénylmaléimide, dans lequel on fait réagir à température élevée de l'anhydride maléique avec de l'aniline en un seul stade, en présence d'un solvant organique non miscible à l'eau capable de former un azéotrope avec l'eau et d'acide p-toluènesulfonique en tant que catalyseur.

Le N-phénylmaléimide est un composé qui possède de nombreuses applications industrielles, notamment comme comonomère améliorant la stabilité thermique de résines acrylonitrile-butadiène-styrène, mieux connues sous la dénomination "ABS".

Il existe déjà différents procédés de fabrication du N-phénylmaléimide. Parmi ces procédés, celui qui a fait l'objet du plus grand nombre de travaux de recherche ces dernières années réalise la production de N-phénylmaléimide en deux stades au départ de l'anhydride maléique et de l'aniline. Dans cette méthode, on fait réagir d'abord l'anhydride maléique avec l'aniline à température modérée pour former de l'acide maléanilique avec un rendement quasi quantitatif, ensuite on soumet l'acide maléanilique, séparé ou non du milieu réactionnel, à une déshydratation thermique à température élevée dans un solvant organique non miscible à l'eau capable de former un azéotrope avec l'eau, tel que le toluène, le xylène, le chlorobenzène et en présence d'un catalyseur acide comme par exemple l'acide sulfurique, phosphorique, etc., l'eau formée au cours de la réaction étant éliminée par distillation azéotropique.

Plus récemment, on a également proposé un procédé de préparation de N-phénylmaléimide par réaction de l'anhydride maléique avec de l'aniline à température élevée en un seul stade. Ici également, la réaction est conduite en présence d'un solvant organique inerte non miscible à l'eau et d'un catalyseur acide; la réaction est effectuée à la température d'ébullition du solvant et l'eau formée au cours de la réaction est éliminée du mélange réactionnel sous forme d'un azéotrope avec le solvant (cf. demande de brevet européen 129.125).

Le procédé en un stade est plus intéressant que le procédé en deux stades étant donné que le procédé en un stade ne requiert pas de quantités importantes de solvant contrairement au procédé en deux stades dans lequel une forte dilution dans le solvant est indispensable pour maintenir en suspension l'acide maléanilique quasi insoluble dans le milieu réactionnel. Le procédé en un stade peut par conséquent être exécuté dans un appareillage moins volumineux et moins coûteux et avec une consommation d'énergie moindre.

Cependant, une difficulté à laquelle aucune méthode connue n'échappe est que la production de N-phénylmaléimide s'accompagne de nombreuses réactions secondaires et conduit inévitablement à l'accumulation d'impuretés indésirables dans le N-phénylmaléimide obtenu. En effet, au cours de la réaction, l'acide maléanilique et l'acide maléique peuvent être produits par hydrolyse respectivement à partir de N-phénylmaléimide et d'anhydride maléique, vu que l'élimination azéotropique de l'eau n'est pas nécessairement instantanée, et le catalyseur acide favorise l'isomérisation de l'acide maléanilique en acide fumaranilique et de l'acide maléique en acide fumarique. En outre, comme le N-phénylmaléimide obtenu au terme de la réaction est thermiquement instable, ce produit se dégrade facilement à température élevée. De plus, sous l'effet du catalyseur acide, l'acide maléanilique a tendance à se condenser avec une autre molécule d'acide maléanilique, avec de l'acide maléique et/ou fumarique pour former des produits de polycondensation de poids moléculaire élevé. Enfin, le N-phénylmaléimide peut également se polymériser par voie radicalaire du fait de la présence d'une double liaison dans la molécule.

On notera que le problème des réactions secondaires et des impuretés se pose encore avec plus d'acuité dans le procédé en un stade que dans le procédé en deux stades vu que dans le procédé en un stade, on part d'anhydride maléique, qui est mis immédiatement en présence du catalyseur acide à une température élevée à laquelle se produit inévitablement une isomérisation partielle d'acide maléique en acide fumarique. C'est pourquoi, il paraît de prime abord encore plus difficile d'obtenir un N-phénylmaléimide de haute pureté dans le procédé en un stade que dans le procédé en deux stades.

Quoi qu'il en soit, dans un cas comme dans l'autre, se pose le problème de la séparation des impuretés du N-phénylmaléimide obtenu au terme de la réaction.
Cette opération est délicate et de plus entraîne inévitablement des pertes en N-phénylmaléimide plus ou moins importantes.

Lorsqu'on examine la littérature dans ce domaine, force est de constater que tous les procédés de récupération et de purification de N-phénylmaléimide décrits jusqu'ici se heurtent à diverses difficultés pratiques; en effet, parmi ces procédés, rares sont ceux qui entrent en ligne de compte pour une application aisée et économique à l'échelle industrielle.

Dans le brevet américain 4.111.879, par exemple, on propose un procédé dans lequel, au terme de la réaction, on distille le solvant, puis on verse le mélange de réaction dans une grande quantité d'eau, on sépare le précipité par filtration et on recristallise le produit souhaité dans l'éthanol.
Si ce procédé devait être appliqué à l'échelle industrielle, il exigerait une consommation d'eau de 17 litres par kilogramme de N-phénylmaléimide produit. En outre, ce procédé conduit forcément à la production simultanée de grandes quantités d'eau contaminée par des impuretés acides diverses, dont il faudra se débarrasser, ce qui soulève des problème écologiques graves.

Il existe des techniques de récupération similaires, mais dans lesquelles la précipitation du produit est réalisée dans un solvant organique, suivie le cas échéant d'une recristallisation dans un autre solvant. Ces procédés amènent évidemment les mêmes problèmes de pollution que ceux signalés plus haut, sans compter qu'il s'ajoute ici le danger d'incendie, d'intoxication et les pertes inévitables en solvants au cours de leur recyclage.

Dans les procédés qui ont recours à une distillation, on a des pertes supplémentaires liées à la décomposition du N-phénylmaléimide et à la formation de produits de polymérisation, qui accompagnent cette opération. En effet, les divers sous-produits acides de même que l'acide utilisé comme catalyseur favorisent la formation de produits de condensation de poids moléculaire élevé au cours de la distillation à température élevée, lesquels non seulement constituent une perte de rendement, mais risquent d'obstruer l'appareillage. Les pertes en N-phénylmaléimide lors de la distillation se chiffrent dans le meilleur des cas à environ 15% en poids, dont 5 à 10% en poids se retrouvent sous forme de polymères et environ 5% à 7% en poids sous forme de produit irrécupérable dans le résidu de distillation.

La demande de brevet européen 165.574 décrit un procédé de fabrication de maléimides en deux stades, dans lequel on propose de purifier les maléimides bruts par un lavage avec de l'eau, suivi d'une distillation en présence d'un stabilisant. Selon une variante préférée, on ajoute aux maléimides bruts un acide organique ou inorganique, par exemple l'acide sulfurique en une quantité d'au moins 1% en poids, basée sur l'acide maléanilique mis en oeuvre, on traite le mélange obtenu à une température comprise entre 5 et 100°C, on sépare une substance résineuse visqueuse qui renferme des sous-produits de la synthèse et on lave la couche organique ainsi traitée avec une quantité d'eau à raison de 1 à 5 fois (en poids) la quantité d'acide maléanilique mis en oeuvre, de façon à débarrasser la couche organique des impuretés solubles dans l'eau. On obtient ainsi des maléimides ne renfermant pratiquement plus d'impuretés, dont on élimine le solvant par distillation. Le N-phénylmaléimide obtenu présente un degré de pureté très élevé (99,5 à 99,8% en poids) et les rendements sont compris entre 70 et 81 moles % basés sur l'acide maléanilique mis en oeuvre (exemples 30 et 32 à 34). Par ce procédé, on parvient donc à séparer efficacement toutes les impuretés et on obtient du N-phénylmaléimide qui présente une très grande pureté. Toutefois, un inconvénient majeur de ce procédé est qu'il nécessite encore toujours le lavage avec des grandes quantités d'eau pour éliminer les impuretés, ce qui entraine inévitablement de graves difficultés techniques et économiques, étant donné que ces eaux contaminées par des produits toxiques ne peuvent pas être rejetées dans l'environnement, et requièrent la construction d'installations d'épuration des eaux, ce qui implique des frais d'investissements supplémentaires considérables, sans compter que les lavages peuvent causer des pertes de N-phénylmaléimide par hydrolyse.

Dans la demande de brevet européen 213.933, on effectue la purification de façon similaire; le mélange de réaction est lavé une ou plusieurs fois à l'eau avant d'entamer la distillation du solvant. Mais contrairement au procédé décrit ci-dessus, la pureté du N-phénylmaléimide obtenu ne dépasse pas 93,6% en poids. En outre, si ce procédé devait être appliqué à l'échelle industrielle, il soulèverait les mêmes problèmes écologiques que le procédé de la demande de brevet européen 165.574.

Enfin, dans la demande de brevet européen 129.125, laquelle se rapporte à la préparation du N-phénylmaléimide en un stade à partir d'anhydride maléique et d'aniline, on ne se préoccupe guère de la purification du produit obtenu. Dans l'exemple 15, le mélange de réaction est concentré par évaporation sous pression réduite, puis soumis à une extraction à l'eau pour éliminer l'acide phosphorique utilisé comme catalyseur, après quoi on soumet le N-phénylmaléimide à une distillation. Cependant, la pureté du produit obtenu n'est pas indiquée.

Il est évident que ce procédé, s'il devait être appliqué à l'échelle industrielle entraînerait les mêmes problèmes écologiques que ceux rappelés plus haut, sans compter que le produit obtenu ne serait pas d'un degré de pureté suffisant, vu que rien n'est prévu pour éliminer les impuretés insolubles dans l'eau, telles que l'acide fumarique, l'acide fumaranilique, etc.

En conclusion, il apparaît que jusqu'à présent on n'a pas encore trouvé de procédé de fabrication de N-phénylmaléimide qui pourrait être appliqué aisément et économiquement à l'échelle industrielle et qui permettrait d'éliminer de manière efficace toutes les impuretés du système, comme par exemple le catalyseur acide et les sous-produits comme l'acide maléique, l'acide fumarique, l'acide maléanilique, l'acide fumaranilique et les produits de polycondensation de poids moléculaire élevé, sans devoir recourir à des traitements qui soulèvent des problèmes écologiques graves et tout en obtenant comme produit du N-phénylmaléimide de très grande pureté avec un rendement éleve.

C'est la résolution de ce problème qui constitue l'objet de la présente invention.

Selon la présente invention, on apporte un procédé amélioré de fabrication de N-phénylmaléimide, dans lequel on fait réagir à température élevée de l'anhydride maléique avec de l'aniline en un seul stade, en présence d'un solvant organique non miscible à l'eau capable de former un azéotrope avec l'eau et d'acide p-toluènesulfonique en tant que catalyseur, et dans lequel on élimine l'eau formée au cours de la réaction sous forme d'un azéotrope avec ledit solvant et on récupère le N-phénylmaléimide à partir du mélange de réaction ainsi obtenu, qui est caractérisé en ce que la récupération du N-phénylmaléimide comporte les stades successifs suivants :
(a) on traite le mélange de réaction dans une zone de dilution et de filtration dans laquelle il est dilué par une quantité supplémentaire dudit solvant, le mélange dilué étant soumis à une filtration à une température comprise entre 20 et 80°C, de préférence environ 50°C, éventuellement sous une pression d'azote de 1 à 5 bars, pour séparer un gâteau solide contenant la totalité du catalyseur et les impuretés, que l'on lave et que l'on écarte du système, et un filtrat liquide consistant en une solution de N-phénylmaléimide dans ledit solvant, et
(b) on soumet la solution de N-phénylmaléimide ainsi obtenue à une distillation sous pression réduite de manière à séparer successivement le solvant et du N-phénylmaléimide à au moins 99% en poids de pureté, qui est recueilli comme produit du procédé, tandis que le résidu de distillation est traité comme indiqué en (a) ci-dessus dans une zone de dilution et de filtration pour en récupérer le N-phénylmaléimide, le filtrat consistant en une solution de N-phénylmaléimide dans le solvant étant renvoyée au stade (a) pour servir à la dilution du mélange de réaction issu d'une synthèse suivante du N-phénylmaléimide,
et en ce que on lave les gâteaux de filtration des stades (a) et (b) avec du solvant obtenu par distillation au stade (b) et on recycle les solvants de lavage au stade de synthèse du N-phénylmaléimide et/ou aux zones de dilution.

Conformément à la présente invention, il a été trouvé qu'il est possible d'effectuer la récupération et la purification du N-phénylmaléimide par un procédé simple, sûr et efficace si l'on se sert de l'acide p-toluènesulfonique en tant que catalyseur pour la synthèse du N-phénylmaléimide à partir de l'anhydride maléique et de l'aniline. En effet, à la différence de la plupart des catalyseurs acides comme l'acide sulfurique, phosphorique, etc., l'acide p-toluènesulfonique utilisé comme catalyseur conformément à la présente invention est soluble dans le milieu de réaction aux températures élevées de la réaction de synthèse du N-phénylmaléimide et solide et insoluble dans le milieu de réaction à basse température. En effet, à l'état anhydride le point de fusion de l'acide p-toluènesulfonique est de 105°C.

C'est pourquoi, il est possible de séparer l'acide p-toluènesulfonique, utilisé comme catalyseur, facilement et intégralement du mélange de réaction par une simple filtration à basse température, après dilution appropriée de ce mélange par le solvant qui a servi comme milieu de réaction.

De plus, selon la présente invention on a fait la découverte surprenante que cette filtration à basse température permet de séparer sous forme de gâteau solide de filtration non seulement la totalité du catalyseur acide, mais également la majeure partie des sous-produits, en particulier l'acide fumarique, l'acide fumaranilique et maléanilique et les produits de polycondensation de poids moléculaire élevé et permet donc d'obtenir un filtrat liquide constitué par une solution de N-phénylmaléimide qui est totalement débarrassée du catalyseur et en grande partie des sous-produits acides, laquelle peut par conséquent être soumise sans inconvénients directement à une distillation sous pression réduite, sans pertes supplémentaires dues à la décomposition du N-phénylmaléimide et sans augmenter de manière significative la formation de polymères qui risquent d'encrasser l'appareillage et de retenir une quantité importante de produit sous forme de résidu de distillation.

Il en résulte qu'au terme de l'opération de distillation, on recueille du N-phénylmaléimide de très grande pureté avec un bon rendement.

Conformément à la présente invention on commence la fabrication du N-phénylmaléimide par sa synthèse qui est réalisée par réaction de l'anhydride maléique avec de l'aniline en un seul stade en présence d'un solvant organique non miscible à l'eau et de l'acide p-toluènesulfonique en tant que catalyseur.

La quantité ajoutée de catalyseur est d'environ 0,5 à environ 4% en poids, de préférence de 1,5 à 2,5% en poids par rapport à la quantité d'anhydride maléique mise en oeuvre. La quantité optimale de catalyseur à mettre en oeuvre peut facilement être déterminée par quelques essais préliminaires.

Le rapport molaire entre l'aniline et l'anhydride maléique dans le procédé conforme à l'invention est avantageusement de 0,90 à 1 et de préférence de 0,94 à 0,98 car il est préférable d'opérer avec un léger excès d'anhydride maléique pour diminuer les réactions secondaires.

La température de la réaction de synthèse du N-phénylmaléimide peut varier entre 110 et 160°C, de préférence de 130 à 150°C.

Comme solvant on utilise dans le procédé conforme à l'invention un solvant organique non miscible à l'eau susceptible de former un azéotrope avec l'eau. Ainsi il est facile d'éliminer l'eau formée en cours de réaction par distillation azéotropique avec le solvant, en conduisant la réaction à la température d'ébullition du mélange réactionnel. Il est souhaitable de choisir un solvant dont la température d'ébullition ne soit pas trop basse et de préférence, supérieure à environ 110°C, pour éviter que la vitesse de la réaction ne soit trop faible. D'un autre côté, la température d'ébullition du solvant ne doit pas non plus être trop élevée ; de préférence, cette température ne dépassera pas 160°C, car les réactions secondaires prennent de plus en plus d'importance à des températures plus élevées. De plus, le solvant devra être un bon solvantdes réactifs et du N-phénylmaléimide, mais un mauvais solvant du catalyseur et des sous-produits de manière à ce qu'on puisse éliminer le catalyseur et la majorité des sous-produits lors de la filtration à froid du mélange de réaction.

Parmi les solvants pouvant entrer en ligne de compte pour les buts de l'invention, on peut citer des hydrocarbures aromatiques chlorés ou non tels que le toluène, les xylènes, les alkyl-benzènes dont les radicaux alkyle renferment de 2 à 3 atomes de carbone et le chlorobenzène, ainsi que des hydrocarbures aliphatiques ou cycloaliphatiques chlorés ou non tels que les triméthylhexanes, l'octane, le nonane, l'éthylcyclohexane, le tétrachloréthane ou des fractions de pétrole bouillant entre 120 et 170°C, et les mélanges des solvants précités.

Il est avantageux de conduire la réaction en présence d'inhibiteurs de polymérisation classiques pour minimiser la polymérisation du N-phénylmaléimide. De tels inhibiteurs sont bien connus dans la technique, et l'on peut citer, à titre d'exemple, des phénols tels que le 2,6-di-tert-butyl-p-crésol,la 2-tert-butyl-hydroquinone, le 4-méthoxyphénol ou le 4-tert-butylcatéchol, des alkyl- et arylphosphites tels que le tributylphosphite, le triphénylphosphite, etc.. Ces inhibiteurs sont ajoutés à la concentration totale de 0,01 à 0,3% en poids, de préférence de 0,02 à 0,1% en poids, par rapport au poids de l'anhydride maléique mis en oeuvre.

La synthèse du N-phénylmaléimide en un stade peut généralement être effectuée de la manière suivante. On dissout l'anhydride maléique dans le solvant, à raison de 15 à 40 parties en poids d'anhydride maléique pour 100 parties en poids du mélange d'anhydride maléique et de solvant, et de préférence à raison de 20 à 30 parties en poids d'anhydride maléique pour 100 parties en poids du mélange d'anhydride maléique et de solvant. On ajoute l'acide p-toluènesulfonique en tant que catalyseur à cette solution dans les proportions indiquées plus haut et on porte le mélange à la température d'ébullition du solvant. Ensuite on introduit progressivement l'aniline dans le mélange réactionnel. Tout au long de la réaction, l'eau formée est éliminée par distillation azéotropique avec le solvant organique.

La durée de la réaction, qui est généralement comprise entre 3 et 5 heures, est déterminée par mesure de la quantité d'eau recueillie.

Selon la présente invention, on récupère ensuite le N-phénylmaléimide à partir du mélange de réaction en deux stades (a) et (b) successifs.

Le stade (a) du procédé conforme à l'invention a pour objet d'éliminer le catalyseur et les divers sous-produits à partir du mélange de réaction.

A cet effet, le mélange de réaction est dilué par le même solvant que celui qui a servi à la réaction, en une quantité représentant 5 à 150% et de préférence 10 à 130% de la quantité de solvant mise en oeuvre au cours de la synthèse. Cette dilution est indispensable pour éviter la précipitation du N-phénylmaléimide, ce qui nuirait au bon fonctionnement du procédé. De plus, comme le N-phénylmaléimide est bon solvant des sous-produits à éliminer, la dilution permet de séparer ces sous-produits en plus grande quantité. Ensuite, on envoie le mélange de réaction dilué dans un appareil de filtration conventionnel et on le filtre à une température comprise entre 20 et 80°C, de préférence environ 50°C, éventuellement sous une pression d'azote de 1 à 5 bars. Le gâteau de filtration, qui contient la totalité du catalyseur acide, une faible quantité de N-phénylmaléimide et la plus grande partie des produits secondaires de la réaction, essentiellement les acides fumaranilique et maléanilique, l'acide fumarique et des produits de polycondensation de poids moléculaire élevé, est lavé avec du solvant obtenu par distillation au stade (b), puis écarté du système en tant que résidu de fabrication. Le filtrat consiste en une solution de N-phénylmaléimide dans le solvant.

Dans le stade (b) du procédé conforme à l'invention, la solution de N-phénylmaléimide obtenue au stade (a) est soumise à une distillation sous pression réduite dans un appareil de distillation conventionnel. Cette opération peut éventuellement être menée en présence d'un inhibiteur de polymérisation tel que mentionné plus haut. On distille le solvant sous une pression de 70 à 150 mbars et à une température de 60 à 100°C, puis on distille le N-phénylmaléimide sous une pression de 6 à 25 mbars et à une température de 160 à 200°C.

Le N-phénylmaléimide recueilli comme fraction principale de cette distillation constitue le produit du présent procédé; sa pureté est d'au moins 99,5% en poids et sa teneur en impuretés est pratiquement nulle. De préférence, on distille d'abord une fraction de tête de N-phénylmaléimide que l'on recycle vers le réacteur pour une synthèse suivante de N-phénylmaléimide.

De son côté, le résidu de distillation est traité exactement comme le mélange de réaction au stade (a), en vue de récupérer le N-phénylmaléimide qu'il contient, c'est-à-dire on le dilue par le même solvant que celui qui a servi à la réaction, dans des proportions qui permettent une filtration aisée, et on envoie la suspension ainsi formée dans un appareil de filtration conventionnel (qui peut être le même que celui utilisé au stade (a)) pour la séparer en un gâteau de filtration solide et un filtrat liquide. Le gâteau, qui contient les impuretés qui n'ont pas été séparées lors de la filtration au stade (a) et une faible proportion de produits formés lors de la distillation, principalement des polycondensats, est lavé, puis écarté du système en tant que résidu de fabrication.

Le filtrat, qui contient du N-phénylmaléimide récupéré en solution dans le solvant, est renvoyé au stade (a) pour servir à la dilution du mélange de réaction issu d'une synthèse suivante de N-phénylmaléimide.

Selon l'invention, on prévoit en outre de recycler intégralement le solvant mis en oeuvre. A cet effet, on lave les gâteaux de filtration des stades (a) et (b) avec du solvant obtenu par distillation au stade (b) et on recycle les solvants de lavage vers le réacteur pour servir de solvant à une nouvelle synthèse de N-phénylmaléimide. Toutefois, on peut également envisager de recycler les solvants de lavage en totalité ou en partie pour servir à la dilution du mélange de réaction au stade (a) et/ou à la dilution du résidu de distillation au stade (b) ou encore selon tout autre schéma qui permet un recyclage convenable du solvant mis en oeuvre.

Les avantages offerts par le procédé de la présente invention sont nombreux :
a) le N-phénylmaléimide obtenu par ce procédé présente une pureté d'au moins 99,5 % en poids, avec une teneur de 0-0,1% en poids d'acide fumarique, 0,04 à 0,1% en poids d'acide et d'anhydride maléique et 0,1 à 0,2 % en poids d'acides maléanilique et fumaranilique;
b) le rendement en N-phénylmaléimide pur est très élevé et atteint au moins 86 moles % par rapport à l'aniline mise en oeuvre;
c) étant donné le degré de pureté très élevé du N-phénylmaléimide obtenu, il reste stable, même sans addition de stabilisants, dans des conditions normales de stockage;
d) pour éliminer les impuretés, on ne doit pas, comme dans certains procédés connus recourir à des lavages avec de grandes quantités d'eau ou à des solvants organiques étrangers à la réaction; on réduit donc drastiquement le problème des eaux résiduaires et de la pollution;
e) à cause du recyclage intégral du solvant dans le système, le seul résidu de fabrication est constitué par les gâteaux de filtration, lesquels renferment la totalité du catalyseur et la majorité des impuretés, ils sont aisément détruits par incinération et le problème de la pollution des eaux est donc réduit au minimum;
f) il peut être exécuté dans des appareils conventionnels peu encombrants;
g) il est simple, sûr et très économique tant du point de vue frais d'investissements que de frais de fonctionnement.

Les exemples donnés ci-après illustrent l'invention sans limiter sa portée.

### Exemple 1.

1. Premier cycle de synthèse et de purification de N-phénylmaléimide.
   Dans un réacteur de 5 litres équipé de moyens d'agitation mécanique et de chauffage, ainsi que d'un condenseur avec séparateur d'eau, on introduit 1440 g de xylène, 600 g (6,12 moles) d'anhydride maléique, 10 g (0,058 mole) d'acide p-toluènesulfonique comme catalyseur, 150 mg de 4-tert-butylcatéchol comme inhibiteur de polymérisation et 100 g de N-phénylmaléimide. On chauffe le mélange sous agitation et on le porte à reflux, puis on introduit en 4 heures 558 g (6 moles) d'aniline, soit un déficit de 2 moles % par rapport à l'anhydride maléique. La réaction se déroule à la température de 145 °C. L'eau formée par la réaction est distillée sous forme d'un azéotrope avec le xylène jusqu'à la fin de la réaction; elle est séparée en continu du xylène, qui est recyclé vers le réacteur.
   Lorsque la réaction est terminée, on dilue le mélange de réaction avec 1800 g de xylène sous agitation. On laisse refroidir le mélange dilué et lorsqu'il atteint une température d'environ 50°C, on le filtre sous pression d'azote.
   Le filtrat, constitué par une solution de N-phénylmaléimide dans le xylène, est soumis à une distillation sous pression réduite. On distille d'abord 3068 g de xylène sous une pression de 100 mbars, à la température de 78°C. Puis, on porte le vide à 13 mbars et on distille à une température de 166-170°C une fraction de tête de N-phénylmaléimide, qui représente 93,4 g (que l'on recycle vers le réacteur pour une synthèse suivante) et ensuite à une température de 171-173°C la fraction principale de N-phénylmaléimide, dont on en recueille 638,2 g. On obtient ainsi, comme produit du procédé, un produit pur dont la teneur en N-phénylmaléimide est au minimum de 99,5% en poids.
   Le gâteau de filtration (115,6 g), qui contient le catalyseur et les impuretés (il s'agit entre autres des divers sous-produits provenant de réactions secondaires), est lavé avec 1750 g de xylène provenant de la distillation précitée, puis il est écarté et peut être brûlé. Le résidu de distillation est dilué sous agitation avec du xylène provenant du lavage du gâteau de filtration et filtré. Le filtrat, qui contient du N-phénylmaléimide récupéré, est recyclé pour servir à la dilution du mélange de réaction issu d'une synthèse suivante, tandis que le gâteau de filtration qui, encore imprégné de xylène représente 150 g, est lavé par 1300 g de xylène provenant de la distillation mentionnée plus haut, puis écarté et brûlé, le xylène de lavage étant recyclé au réacteur pour servir de solvant à une nouvelle synthèse de N-phénylmaléimide.
2. Deuxième cycle de synthèse et de purification du N-phénylmaléimide.
   Le réacteur contient déjà 1269 g de xylène et 93,4 g de fraction de tête de N-phénylmaléimide provenant du premier cycle de synthèse. On y ajoute 171 g de xylène frais pour compenser la perte de xylène. On ajoute au réacteur 600 g (6,12 moles) d'anhydride maléique, 10 g (0,058 mole) d'acide p-toluènesulfonique et 150 mg de 4-tert-butylcatéchol. On procède ensuite comme au premier cycle de synthèse. On porte le mélange à reflux, on y ajoute 558 g (6 moles) d'aniline et lorsque la réaction est terminée, on dilue le mélange de réaction sous agitation avec le solde du xylène provenant du premier cycle de synthèse, qui atteint 1800 g de xylène. On filtre le mélange dilué et on distille le filtrat, ce qui permet de recueillir 2950 g de xylène, 115,9 g de fraction de tête de N-phénylmaléimide (que l'on recycle au réacteur pour une troisième synthèse) et 814,4 g d'une fraction principale de N-phénylmaléimide. On lave alors le gâteau de filtration avec du xylène distillé recueilli ci-dessus, puis il est écarté et peut être brûlé.
   Le résidu de distillation est dilué avec du xylène ayant servi au lavage du gâteau de filtration, le filtrat est recyclé pour servir à la dilution du mélange de réaction issu d'une troisième synthèse, tandis que le gâteau de filtration est lavé par du xylène distillé recueilli ci-dessus, et le xylène de lavage est recyclé au réacteur pour servir de solvant pour un cycle ultérieur de synthèse de N-phénylmaléimide.
3. Troisième cycle de synthèse et de purification du N-phénylmaléimide et cycles suivants.
   En opérant exactement comme décrit au point 1 ci-dessus, on procède encore à 10 cycles successifs de synthèse et de purification de N-phénylmaléimide. Pour chaque cycle de synthèse, les quantités de réactifs, de fraction de tête de N-phénylmaléimide (PMI) recueillie et recyclée, de catalyseur, d'inhibiteur de polymérisation, de même que la quantité de N-phénylmaléimide pur recueilli comme fraction principale, sont reportés dans le tableau I.

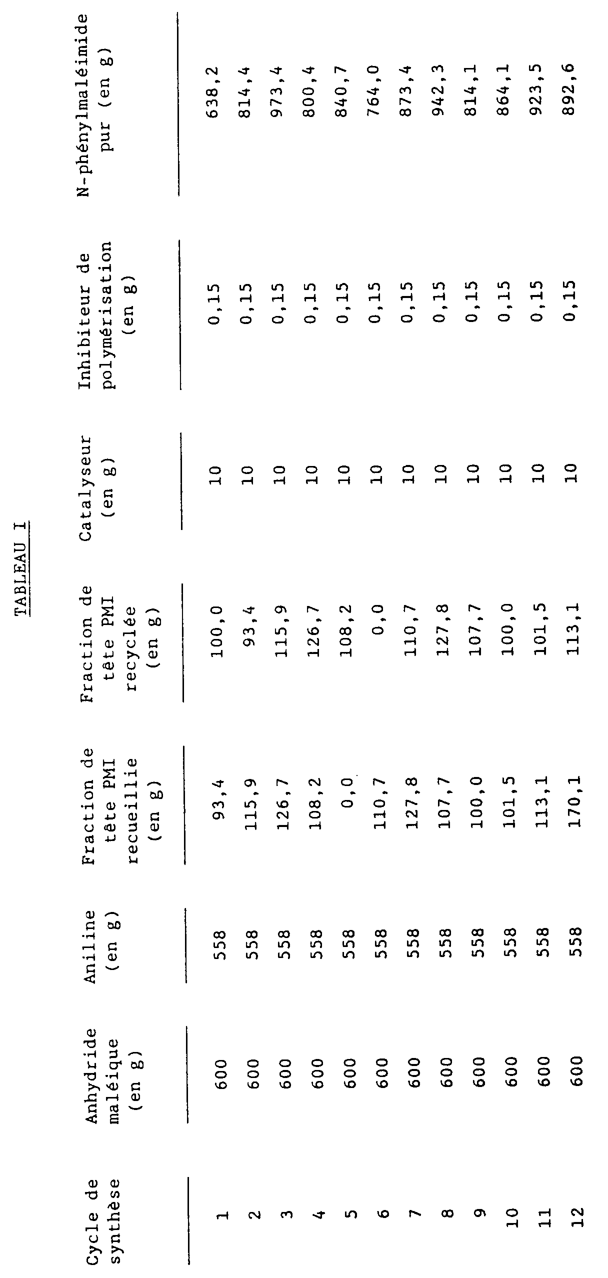

Après 12 cycles de synthèse, on obtient donc 10.141,1 g de N-phénylmaléimide comme fraction principale. On récupère encore 170,1 g de N-phénylmaléimide dans la dernière fraction de tête, 107,4 g dans le xylène de lavage, 9,0 g dans le xylène distillé et 389,9 g dans le filtrat du résidu de distillation, ce qui donne un total de 10.817,5 g de N-phénylmaléimide. Soustraction faite des 100 g de N-phenylmaléimide introduits lors du premier cycle, on obtient un rendement global de 86,1 moles % de N-phénylmaléimide par rapport à l'aniline mise en oeuvre.

La teneur en N-phénylmaléimide du produit pur ainsi obtenu est de 99,5% en poids; il contient moins de 0,1% en poids d'acide fumarique, 0,04 à 0,1% en poids d'acide et d'anhydride maléique, 0,1 à 0,2% en poids d'acides maléanilique et fumaranilique et moins de 0,1% en poids de composés indéterminés à poids moléculaire élevé.

### Exemple 2 (comparatif). Préparation et purification de N-phénylmaléimide sans filtration préalable.

Dans cet exemple comparatif, on procède directement à la distillation du mélange de réaction, sans élimination préalable par filtration, du catalyseur et des sous-produits acides provenant des réactions secondaires.

Dans un réacteur d'une capacité de 5 litres muni de moyens de chauffage et d'agitation mécanique et d'un condenseur avec séparateur d'eau, on introduit 1440 g de xylène, 600 g (6,12 moles) d'anhydride maléique, 10 g (0,058 mole) d'acide p-toluènesulfonique comme catalyseur, 150 mg de 4-tert-butylcatéchol comme inhibiteur de polymérisation et 100 g de N-phénylmaléimide. On chauffe le mélange sous agitation et on le porte à reflux, puis on introduit en 4 heures 558 g (6 moles) d'aniline, soit un déficit de 2 moles % par rapport à l'anhydride maléique. La réaction se déroule à la température de 145°C. L'eau formée au cours de la réaction est distillée sous forme d'un azéotrope avec le xylène jusqu'à la fin de la réaction.

Lorsque la réaction est terminée, le mélange de réaction est soumis à une distillation sous pression réduite. On distille d'abord le xylène (1397 g) sous une pression de 100 mbars et à une température de 78°C. On porte ensuite le vide à 13 mbars et on distille 109 g d'une fraction de tête de N-phénylmaléimide, dont la teneur en N-phénylmaléimide est de 96,5% en poids, à une température de 166-170°C. Cette fraction de tête peut être utilisée dans une autre synthèse de N-phénylmaléimide. On distille ensuite la fraction principale de N-phénylmaléimide dont on recueille en laboratoire des quantités extrêmement variables (environ 600 g dans cet exemple précis).

On obtient un produit, dont la teneur en N-phénylmaléimide est, au mieux, de 98,55 % en poids. Ce produit de qualité nettement inférieure à celle obtenue dans l'exemple 1 contient, comme impuretés principales, 0,7% en poids d'acide fumarique, 0,3% en poids d'acide et d'anhydride maléique et 0,3% en poids d'acide fumaranilique.

Par ailleurs, il subsiste en quantités très variables, un résidu de distillation au fond de l'appareil de distillation. Ce résidu est constitué d'une masse foncée dont il est impossible d'extraire le N-phénylmaléimide qu'il contient. D'après l'analyse par chromatographie en phase gazeuse (GC) et par chromatographie en phase liquide à haute performance (HPLC), ce résidu de distillation contient, en poids, 25 % de N-phénylmaléimide irrécupérable, 0,6 % d'acide et d'anhydride maléique, 1,4 % d'acide fumarique, 5 % d'acide fumaranilique et 68 % de résidu (polycondensats, polymères et catalyseur).
Extrapolé en stade pilote, le procédé décrit dans l'exemple 2 a même conduit à des rendements en N-phénylmaléimide voisin de zéro, suite à la polymérisation en masse du mélange réactionnel en cours de distillation.

Cet exemple comparatif montre clairement que la présence du catalyseur et des sous-produits acides dans le mélange de réaction favorise considérablement la formation de produits de polycondensation de poids moléculaire élevé au cours de la distillation et contribue à obtenir une qualité de N-phénylmaléimide inférieure.

En effet, lorsqu'on compare les exemples 1 et 2, on constate que si l'on procède directement à la distillation du mélange de réaction sans filtration préalable de celui-ci :
- il y a une perte importante de produit utile et on recueille un N-phénylmaléimide impur (pureté de 98,55% en poids) avec un rendement très aléatoire vu les risques de polymérisation en cours de distillation;
- le résidu qui se forme dans l'appareil de distillation contient une quantité considérable de polycondensats et en outre du N-phénylmaléimide qu'il n'est plus possible d'extraire.
Le procédé selon la présente invention permet au contraire une récupération aisée et presque complète du N-phénylmaléimide à partir du mélange de réaction sans formation significative de produits de polycondensation au cours de la distillation. On obtient un produit de haute pureté dont la teneur en N-phénylmaléimide est supérieure ou égale à 99,5 % en poids et dont la teneur en impuretés est pratiquement nulle, avec un rendement global qui dépasse 86 moles %. En outre, le N-phénylmaléimide qui est retenu dans le résidu de distillation est aisément récupérable.

### Exemple 3. Effets de la filtration sur la composition du N-phénylmaléimide.

On montre dans cet exemple les effets de la filtration du mélange de réaction sur la composition du N-phénylmaléimide brut (non distillé). A cet effet, on a rassemblé au tableau IL ci-dessous, les données suivantes obtenues par analyse GC et HPLC où la composition
- A est la composition d'un N-phénylmaléimide brut, obtenu dans le procédé de l'exemple 1, avant sa distillation, c'est-à-dire après avoir dilué et filtré le mélange de réaction et distillé le xylène et la fraction de tète (ci-après dénommé produit A) ;
- B est la composition d'un N-phénylmaléimide brut, obtenu dans le procédé de l'exemple 2, avant sa distillation, c'est-à-dire après avoir distillé le xylène et la fraction de tète (sans filtration du mélange réactionnel ; ci-après dénommé produit B, non conforme à l'invention).

**TABLEAU II**

| Composition du N-phénylmaléimide brut (en % en poids) | | |
|---|---|---|
| | A (avec filtration) | B (comparatif) (sans filtration) |
| N-phénylmaléimide | 91,9 | 88,6 |
| Acide p-toluènesulfonique | - | 0,9 |
| Acide fumarique | 0,03 | 0,9 |
| Acide et anhydride maléique | 0,36 | 0,82 |
| Acides fumaranilique et maléanilique | 0,32 | 1,3 |
| Polycondensats et polymères | 7,39 | 7,48 |

Ce tableau montre clairement que le N-phénylmaléimide brut obtenu selon le procédé de la présente invention (produit A) est beaucoup plus pur que le N-phénylmaléimide brut qui n'est pas obtenu selon le procédé de la présente invention, c'est-à-dire sans dilution et filtration (produit B). On voit en particulier que la teneur en acides fumarique, fumaranilique et maléanilique du produit B est beaucoup plus élevée.

On voit aussi que le N-phénylmaléimide brut obtenu dans le procédé de l'exemple 2 (produit B non conforme à l'invention) contient de surcroît une quantité importante du catalyseur acide, alors que le N-phénylmaléimide brut de l'exemple 1 conforme à l'invention (produit A) en est complètement débarrassé.

A titre indicatif, on donne au tableau III ci-dessous la composition d'un gâteau de filtration obtenu dans l'exemple 1 après la filtration du mélange de réaction dilué.

**TABLEAU III**

| Composition du gâteau de filtration sec de l'exemple 1 (en % en poids) | |
|---|---|
| N-phénylmaléimide | 3,3 |
| Acide p-toluènesulfonique | 14,8 |
| Acide fumarique | 7,9 |
| Acide et anhydride maléique | 3,2 |
| Acides fumaranilique et maléanilique | 35,9 |
| Polycondensats et polymères | 34,9 |

On voit que le gâteau de filtration retient non seulement le catalyseur, mais également la plus grande partie des sous-produits, en particulier l'acide fumarique, l'acide fumaranilique et maléanilique et les produits de polycondensation de poids moléculaire élevé.

On notera que la faible quantité de N-phénylmaléimide contenue dans le gâteau peut être récupérée par lavage avec le xylène ayant servi à la synthèse et recyclée.

### Exemple 4. Stabilité thermique du N-phénylmaléimide.

a) Stabilité du N-phénylmaléimide brut avant distillation.
   Dans un premier essai, on a comparé la stabilité thermique du N-phénylmaléimide brut obtenu dans le procédé de l'exemple 1 conforme à l'invention (produit A de l'exemple 3) avec la stabilité thermique du N-phénylmaléimide brut obtenu dans le procédé de l'exemple 2 non conforme à l'invention (produit B de l'exemple 3). Avant l'essai on a ajouté au produit B, 100 ppm d'éther monométhylique de l'hydroquinone, tandis que le produit A a été utilisé tel quel. Lorsqu'on place des échantillons des produits A et B à 120, 135 et 150°C, on fait après une semaine les constatations suivantes : les échantillons du produit A ont légèrement bruni, leur viscosité reste inchangée et on ne décèle aucun changement de composition par chromatographie en phase liquide à haute performance (HPLC); par contre, dans les échantillons du produit B, un précipité impossible à dissoudre apparaît à 120 et 135°C, et à 150°C, l'échantillon du produit B est insoluble dans l'éluant, rendant son analyse par HPLC impossible.
   Dans un autre essai de stabilité réalisé aux mêmes températures de 120, 135 et 150°C, on a comparé le produit A pur avec le produit A auquel on a ajouté respectivement 1 et 5 % en poids de gâteau de filtration obtenu dans l'exemple 1 après la filtration du mélange de réaction dilué.
   Après 9 jours, on constate que le produit A pur présente une viscosité inchangée, alors que les échantillons contenant du gâteau de filtration présentent une viscosité qui augmente progressivement au cours du temps, d'autant plus rapidement que le pourcentage de gâteau ajouté et la température sont élevés.
   Il ressort de ces essais que le N-phénylmaléimide brut obtenu selon le procédé de la présente invention est remarquablement stable, ce qui permet, en cas de besoin, l'entreposage du produit brut à l'état liquide sans perte sensible, par exemple dans l'attente d'une opération de distillation.
   Par contre, la présence des acides organiques issus des réactions secondaires est préjudiciable pour la stabilité du N-phénylmaléimide en provoquant la formation de produits de polymérisation.
b) Stabilité du N-phénylmaléimide pur (distillé).
   Lorsqu'on maintient des échantillons du N-phénylmaléimide pur obtenu selon l'exemple 1 à la température de 90°C, on constate qu'après 13 jours ces échantillons ne présentent pas de changement d'aspect, ni de composition par analyse en chromatographie en phase gazeuse(GC). Lorsqu'on les place à des températures plus élevées (120, 150 et 180 °C), on observe après 3 jours un léger brunissement des échantillons, mais aucun changement de composition n'est perceptible par HPLC et GC.
   Le N-phénylmaléimide pur obtenu selon l'invention est donc remarquablement stable, même en l'absence d'inhibiteurs de polymérisation. Dès lors, on peut éventuellement envisager un stockage sans addition de stabilisants, ce qui peut constituer un avantage non négligeable dans certaines applications particulières.

## Revendications

1. Procédé de fabrication de N-phénylmaléimide, dans lequel on fait réagir à température élevée de l'anhydride maléique avec de l'aniline en un seul stade, en présence d'un solvant organique non miscible à l'eau capable de former un azéotrope avec l'eau et d'acide p-toluènesulfonique en tant que catalyseur, et dans lequel on élimine l'eau formée au cours de la réaction sous forme d'un azéotrope avec ledit solvant et on récupère le N-phénylmaléimide à partir du mélange de réaction ainsi obtenu, caractérisé en ce que la récupération du N-phénylmaléimide comporte les stades successifs suivants:
(a) on traite le mélange de réaction dans une zone de dilution et de filtration dans laquelle il est dilué par une quantité supplémentaire dudit solvant, le mélange dilué étant soumis à une filtration à une température comprise entre 20 et 80°C, de préférence environ 50°C, éventuellement sous une pression d'azote de 1 à 5 bars, pour séparer un gâteau solide contenant la totalité du catalyseur et les impuretés, que l'on lave et que l'on écarte du système, et un filtrat consistant en une solution de N-phénylmaléimide dans ledit solvant, et
(b) on soumet la solution de N-phénylmaléimide ainsi obtenue à une distillation sous pression réduite de manière à séparer successivement le solvant et du N-phénylmaléimide à au moins 99 % de pureté, qui est recueilli comme produit du procédé, tandis que le résidu de distillation est traité comme indiqué en (a) ci-dessus dans une zone de dilution et de filtration pour en récupérer le N-phénylmaléimide, le filtrat consistant en une solution de N-phénylmaléimide dans le solvant étant renvoyée au stade (a) pour servir à la dilution du mélange de réaction issu d'une synthèse suivante du N-phénylmaléimide,
et en ce qu'on lave les gâteaux de filtration des stades (a) et (b) avec du solvant obtenu par distillation au stade (b) et on recycle les solvants de lavage au stade de synthèse du N-phénylmaléimide et/ou aux zones de dilution.

2. Procédé selon la revendication 1, caractérisé en ce que la réaction est effectuée à une température comprise entre 110 et 160°C, de préférence entre 130 et 150°C.

3. Procédé selon l'une quelconque des revendications 1 et 2, caractérisé en ce que la quantité utilisée d'acide p-toluènesulfonique est comprise entre environ 0,5 et environ 4% en poids, de préférence entre entre 1,5 et 2,5% en poids, par rapport à la quantité d'anhydride maléique mise en oeuvre.

4. Procédé selon l'une quelconque des revendications 1 à 3, caractérisé en ce que le rapport molaire entre l'aniline et l'anhydride maléique est compris entre 0,90 et 1, de préférence entre 0,94 et 0,98.

5. Procédé selon l'une quelconque des revendications 1 à 4, caractérisé en ce que l'on utilise comme solvant un hydrocarbure aromatique chloré ou non, un hydrocarbure aliphatique ou cycloaliphatique chloré ou non ou un mélange des solvants précités.

6. Procédé selon la revendication 5, caractérisé en ce que le solvant est du xylène.

7. Procédé selon l'une quelconque des revendications 1 à 6, caractérisé en ce que, au stade (a), le mélange de réaction est dilué par ledit solvant en une quantité représentant 5 à 150% et de préférence 10 à 130% de la quantité de solvant mise en oeuvre au cours de la synthèse.

8. Procédé selon l'une quelconque des revendications 1 à 7, caractérisé en ce que, au stade (b), on distille le solvant sous une pression de 70 à 150 mbars et à une température de 60 à 100°C.

9. Procédé selon l'une quelconque des revendications 1 à 8, caractérisé en ce que, au stade (b), on distille d'abord une fraction de tête de N-phénylmaléimide, que l'on recycle au stade de synthèse du N-phénylmaléimide.

10. Procédé selon l'une quelconque des revendications 1 à 9, caractérisé en ce que, au stade (b), le N-phénylmaléimide est recueilli comme fraction principale par distillation sous une pression de 6 à 25 mbars et à une température de 160 à 200°C.

## Claims

1. A process for the manufacture of N-phenylmaleimide in which maleic anhydride is reacted with aniline in a single step at elevated temperature in the presence of a water-immiscible organic solvent capable of forming an azeotrope with water and of p-toluenesulfonic acid as catalyst, and in which the water formed during the reaction is removed in the form of an azeotrope with the said solvent, and the N-phenylmaleimide is recovered from the reaction mixture thus obtained, characterised in that recovery of the N-phenylmaleimide comprises the successive steps of:
(a) treating the reaction mixture in a dilution and filtration zone, in which it is diluted with an additional amount of the said solvent, the diluted mixture being filtered at a temperature comprised between 20 and 80°C, preferably about 50°C, optionally under a Nitrogen pressure of 1 to 5 bars, in order to separate a solid cake containing the whole of the catalyst and impurities, which cake is washed and removed from the system, and a liquid filtrate consisting of a solution of N-phenylmaleimide in said solvent, and
(b) distilling the solution of N-phenylmaleimide thus obtained under reduced pressure so as to successively separate the solvent and N-phenylmaleimide with a purity of at least 99%, which is recovered as the product of the process, whereas the distillation residue is treated as indicated in (a) above in a dilution and filtration zone in order to recover the N-phenylmaleimide therefrom, the filtrate consisting of a solution of N-phenylmaleimide in the solvent being sent back to step (a) in order to dilute the reaction mixture obtained from a subsequent synthesis of N-phenylmaleimide
and in that the filter cakes from steps (a) and (b) are washed with solvent obtained by distillation in step (b) and in that the resulting wash solvents are recycled for use in the reaction stage and/or dilution zones.

2. A process according to claim 1, characterised in that the reaction is carried out at a temperature comprised between 110 and 160°C, preferably between 130 and 150°C.

3. A process according to claims 1 or 2, characterised in that the amount of p-toluenesulfonic acid used is comprised between about 0.5 to about 4% by weight, preferably between 1.5 and 2.5 % by weight with respect to the amount of maleic anhydride used.

4. A process according to any of claims 1 to 3, characterised in that the molar ratio of aniline to maleic anhydride is comprised between 0.90 and 1, preferably between 0.94 and 0.98.

5. A process according to any of claims 1 to 4, characterised in that the solvent used is a chlorinated or non-chlorinated aromatic hydrocarbon, a chlorinated or non-chlorinated aliphatic or cycloaliphatic hydrocarbon or a mixture of the aforesaid solvents.

6. A process according to claim 5, characterised in that the solvent is xylene.

7. A process according to any of claims 1 to 6, characterised in that, in step (a), the reaction mixture is diluted with the said solvent in an amount representing 5 to 150% and preferably 10 to 130% of the amount of solvent used in the course of the synthesis.

8. A process according to any of claims 1 to 7, characterised in that, in step (b), the solvent is distilled off under a pressure of 70 to 150 mbars and at a temperature of 60 to 100°C.

9. A process according to any of claims 1 to 8, characterised in that, in step (b), a head fraction of N-phenylmaleimide is first distilled off and recycled to the stage of N-phenylmaleimide synthesis.

10. A process according to any of claims 1 to 9, characterised in that, in step (b), the N-phenylmaleimide is recovered as the main fraction by distillation under a pressure of 6 to 25 mbars and at a temperature of 160 to 200°C.

## Patentansprüche

1. Verfahren zur Herstellung Von N-Phenylmaleinimid, bei dem man Maleinsäureanhydrid mit Anilin in einer einzigen Stufe in Gegenwart eines organischen, mit Wasser nicht mischbaren Lösungsmittels, das in der Lage ist, mit Wasser ein Azeotrop zu bilden, und p-Toluolsulfonsäure als Katalysator bei erhöhter Temperatur umsetzt und bei dem man das im Verlauf der Reaktion gebildete Wasser, in Form eines Azeotrops mit besagtem Lösungsmittel entfernt und man das N-Phenylmaleinimid aus dem so erhaltenen Reaktionsgemisch gewinnt, dadurch gekennzeichnet, daß die Gewinnung des N-Phenylmaleinimids die folgenden aufeinanderfolgenden Stufen umfaßt:
(a) man behandelt das Reaktionsgemisch in einer Verdünnungs- und Filtrationszone, in der es durch eine zusätzliche Menge besagten Lösungsmittels verdünnt wird, wobei das verdünnte Gemisch bei einer Temperatur zwischen 20 und 80 °C, vorzugsweise etwa 50 °C, gegebenenfalls unter einem Stickstoffdruck von 1 bis 5 bar, filtriert wird, um einen festen den gesamten Katalysator und die Verunreinigungen enthaltenden Kuchen, den man wäscht und den man aus dem System entfernt, und ein Filtrat, das aus einer Lösung von N-Phenylmaleinimid in besagtem Lösungsmittel besteht, abzutrennen, und
(b) man destilliert die so erhaltene N-Phenylmaleinimid-Lösung unter vermindertem Druck, um nacheinander das Lösungsmittel und N-Phenylmaleinimid, das als Produkt des Verfahrens gewonnen wird, mit wenigstens 99% Reinheit abzutrennen, während der Destillationsrückstand, wie oben in (a) angegeben, in einer Verdünnungs- und Filtrationszone behandelt wird, um daraus das N-Phenylmaleinimid zu gewinnen, wobei das Filtrat aus einer Lösung von N-Phenylmaleinimid in dem Lösungsmittel besteht, das in die Stufe (a) zurückgeschickt wird, um zur Verdünnung des aus einer folgenden Synthese des N-Phenylmaleinimids stammenden Reaktionsgemischs zu dienen,
und dadurch, daß man die Filterkuchen der Stufen (a) und (b) mit Lösungsmittel wäscht, das durch Destillation in Stufe (b) erhalten wurde, und man die Waschlösungsmittel in die Synthesestufe des N-Phenylmaleinimids und/oder in die Verdünnungszonen zurückführt.

2. Verfahren gemäß Anspruch 1, dadurch gekennzeichnet, daß die Reaktion bei einer Temperatur zwischen 110 und 160 °C, vorzugsweise zwischen 130 und 150 °C ausgeführt wird.

3. Verfahren gemäß einem der Ansprüche 1 und 2, dadurch gekennzeichnet, daß die verwendete Menge an p-Toluolsulfonsäure, bezogen auf die eingesetzte Menge an Maleinsäureanhydrid, zwischen etwa 0,5 und etwa 4 Gew.-%, vorzugsweise zwischen1,5 und 2,5 Gew.-%, liegt.

4. Verfahren gemäß einem der Ansprüche 1 bis 3, dadurch gekennzeichnet, daß das Molverhältnis zwischen dem Anilin und dem Maleinsäureanhydrid zwischen 0,90 und 1, vorzugsweise zwischen 0,94 und 0,98, liegt.

5. Verfahren gemäß einem der Ansprüche 1 bis 4, dadurch gekennzeichnet, daß man als Lösungsmittel einen chlorierten oder nicht chlorierten aromatisohen Kohlenwasserstoff, einen chlorierten oder nicht chlorierten aliphatischen oder cycloaliphatischen Kohlenwasserstoff oder ein Gemisch der oben erwähnten Lösungsmittel verwendet.

6. Verfahren gemäß Anspruch 5, dadurch gekennzeichnet, daß das Lösungsmittel Xylol ist.

7. Verfahren gemäß einem der Ansprüche 1 bis 6, dadurch gekennzeichnet, daß das Reaktionsgemisch in Stufe (a) mit besagtem Lösungsmittel in einer Menge verdünnt wird, die 5 bis 150% und vorzugsweise 10 bis 130% der im Verlauf der Synthese eingesetzten Lösungsmittelmenge darstellt.

8. Verfahren gemäß einem der Ansprüche 1 bis 7, dadurch gekennzeichnet, daß man das Lösungsmittel in Stufe (b) unter einem Druck von 70 bis 150 mbar und bei einer Temperatur von 60 bis 100 °C destilliert.

9. Verfahren gemäß einem der Ansprüche 1 bis 8, dadurch gekennzeichnet, daß man in Stufe (b) zuerst eine Kopffraktion von N-Phenylmaleinimid destilliert, die man in die Synthesestufe des N-Phenylmaleinimids zurückführt.

10. Verfahren gemäß einem der Ansprüche 1 bis 9, dadurch gekennzeichnet, daß das N-Phenylmaleinimid in Stufe (b) durch Destillation unter einem Druck von 6 bis 25 mbar und bei einer Temperatur von 160 bis 200 °C als Hauptfraktion gewonnen wird.
